# EUROPÄISCHE PATENTANMELDUNG

(11) **EP 1 491 143 A1**
(43) Veröffentlichungstag der Anmeldung: **29.12.2004**
(21) Anmeldenummer: 04090261.1
(22) Anmeldetag: 28.06.2004
(51) Int. Cl.: A61B 5/15

(54) **Diagnostisches oder analytisches Einmalgebrauchsmittel mit integrierter Lanzette**

(30) Priorität: 27.06.2003 DE 10329980; 25.09.2003 DE 10345663
(71) Anmelder: SensLab - Gesellschaft zur Entwicklung und Herstellung bioelektrochemischer Sensoren mbH, 04347 Leipzig (DE)
(72) Erfinder: Gründig, Bernd, Dr., 04318 Leipzig (DE)
(74) Vertreter: Rasch, Dorit

(57) **Zusammenfassung**

Es wird ein Einmalgebrauchsmittel vorgeschlagen, umfassend einen Sensor (11) und eine integrierte Lanzette für eine Lanzettierung von Haut zur Gewinnung einer Körperflüssigkeit, wobei die Lanzette einen kegelförmigen, formschlüssig angeordneten Dorn (1) umfasst, der auf einer unter Druckbeaufschlagung sprunghaft und reversibel verformbaren Materialauswölbung (3a,c,d,f) positioniert ist und mindestens eine die Körperflüssigkeit aufnehmende, mit einer Reaktionsschicht des Sensors wirkverbindbare Nut (6) und/oder einen Kanal (7) aufweist.

## Beschreibung

Die Erfindung betrifft ein diagnostisches oder analytisches Einmalgebrauchsmittel zur Gewinnung einer Körperflüssigkeit mittels einer Lanzettierung von Haut sowie ein Verfahren zur Herstellung des Einmalgebrauchsmittels und die Verwendung des Einmalgebrauchsmittels für die Lanzettierung der Haut, der Abnahme von Körperflüssigkeit sowie der Bestimmung der Glucose- und/oder Laktatwerte in der Körperflüssigkeit.

Einmalgebrauchssensoren in Kombination mit miniaturisierten Handmessgeräten sind im Point of Care Bereich insbesondere bei der Diabetesüberwachung üblich, um schnell und einfach die Kontrolle des Glucosegehaltes im Blut auch durch den analytischen Laien zu ermöglichen. Eine Reihe unterschiedlicher Systeme beispielsweise von Roche, Schweiz, Johnson & Johnson, USA, Abbott, USA und Bayer, USA sind am Markt etabliert. Bei den analytischen Elementen bzw. Einmalgebrauchssensoren handelt es sich um Systeme im sogenannten Teststrip-Format, die eine Oxidoreduktase in Kombination mit einem Redoxmediator oder Redoxfarbstoff nutzen, um Glucose selektiv zu detektieren.

Die Signalwandlung zu einem quantifizierbaren elektrischen Messsignal erfolgt reflektometrisch oder mittels elektrochemischer Transducer. Die Analysenzeit beträgt bei den bekannten kommerzialisierten Glucosesensoren zwischen 5 sec und 20 sec. Für die Messung sind nur noch wenige Mikroliter Blut, bei einem bekannten kommerzialisierten Einwegsensor weniger als ein Mikroliter erforderlich. Das erforderliche Probevolumen gelangt bei neueren Glucoseeinwegsensoren mittels Kapillarkraftwirkung definiert in die Reaktionsschicht oder Reaktionskammer, so dass in der Regel eine gute Reproduzierbarkeit der Messung gesichert wird.

Vor der schnellen und mit geringstem Probevolumina auskommenden Messung, steht jedoch die Probegewinnung, die in einem ersten Schritt die Erzeugung eines Bluttropfens auf einer Fingerbeere oder am Ohrläppchen und im Anschluss die Überführung eines Teils des Bluttropfens auf bzw. in den Sensor erfordert. Vor allem die Erzeugung eines Bluttropfens durch den analytischen Laien erfordert den Umgang mit einer Lanzettierhilfe und Lanzetten, der vergleichsweise umständlich ist. Die verwendeten Lanzettenspitzen erzeugen aufgrund ihrer Materialstärke und der mehr oder weniger gut ausgebildeten Spitzen immer noch ein Schmerzempfinden während der Lanzettierung. Darüber hinaus ist für den regelmäßigen Wechsel der Lanzette zu sorgen. Schließlich muss die Entsorgung der Lanzetten derart sein, dass keine Gefahr gegen Dritte durch die mit Blut kontaminierten Spitzen ausgeht.

Die Probegewinnung und deren Ausmessung ist daher in neueren Systemen integriert, um sie in einem Schritt durchzuführen oder zumindest Lanzette und Sensor in einer Einheit zu realisieren und neue Wege für eine weniger schmerzhafte Lanzettierung zu gehen. Dazu werden prinzipiell zwei technische Lösungsansätze verfolgt:
(1) Der erste Ansatz sieht die Verwendung einer Vorrichtung vor, in der sowohl die austauschbare Lanzette als auch der Einmalgebrauchssensor separat enthalten sind und über eine interne Aktorik nacheinander oder parallel geräteintern appliziert werden. Die Signalverarbeitung und -auswertung ist ebenfalls in der Vorrichtung integriert. Beispielsweise enthält eine bekannte zylinderförmige Vorrichtung (US 5,951,492) jeweils austauschbar eine Lanzette, ein Kapillarröhrchen und einen Einmalgebrauchssensor. Nach Aufsetzen der Stirnfläche der Vorrichtung auf eine Hautpartie wird mittels Federmechanismus durch Einstechen der Lanzette in die Haut ein Bluttropfen generiert, der über das Kapillarröhrchen zur Reaktionsschicht des Sensors gelangt und dort auf den Zielparameter quantifiziert wird.
   Ähnliche Vorrichtungen sehen ein austauschbares analytisches Element und eine Lanzette in einer Anordnung derart vor, dass das nach dem Einstechvorgang austretende Blut über eine Kapillare oder saugfähiges Dochtmaterial direkt zur Reagenzschicht des Einmalgebrauchssensors geleitet wird (DE 10101658, US 2002/0198444, US6014577). Weitere bekannte technische Lösungen enthalten zusätzlich eine Vakuumerzeugung unmittelbar an dem lanzettierten Hauthabschnitt, um den Bluttropfen schneller zu generieren (US 2002/0130042, US 2002/0169393) oder enthalten stapelbare oder revolverartig angeordnete Sensorelemente, mit denen die Vorrichtung für eine bestimmte Anzahl von Messungen vormagaziniert wird (DE 10101658, US 2002/0169393).
(2) Der zweite Ansatz zielt auf eine direkte Integration von Sensor und Lanzette.
   In zwei bekannten technischen Lösungen (DE 10142 232, EP 1285 629) werden Lanzetten unmittelbar unter oder seitlich vom Sensorelement angeordnet. Beide bilden eine Einmalgebrauchseinheit. Die Stechbewegung der Lanzette, die unabhängig vom Sensor beweglich ist, erfolgt über eine Aktorik im Signalverarbeitungs- und Auslesegerät. Die Probeübertragung von der Einstichstelle zum Sensorelement wird mittels kapillarer Kanäle realisiert. Darüber hinaus sind technische Lösungen bekannt, bei denen mit Hilfe der Mikro- bzw. Nanosystemtechnik Lanzettenspitzen aus Siliziumchips strukturiert werden, die bereits das analytische Element enthalten (US 2002/0177763, US 2002/0160520) oder gegebenenfalls eine separate Lanzettierspitze in unmittelbarer Nähe des mikrostrukturierten Sensorelements aufweisen (US 2002/0177761). Die Aktorik zum Lanzettieren erfolgt über eine Vorrichtung, die Bestandteil des Signalverarbeitungs- und Anzeigegerätes ist.
   In einer anderen technischen Lösung (US 2002/0177788) wird ein Sensor beschrieben, dessen kapillare Lanzettenspitze über eine Vorkammer, mit deren Hilfe ein Unterdruck erzeugt werden kann, mit der Messkammer des Sensors in Verbindung steht.
   Eine weitere bekannte technische Lösung (US 6132449) nutzt eine oder mehrere federnd im Sensorelement fixierte Lanzettenspitzen. Nach Auflegen des Sensorelements auf die Hautoberfläche penetriert die Lanzettenspitze durch Entspannung der Zugfedern die Haut. Der resultierende Bluttropfen wird über Kapillarkräfte zur Indikationsfläche des Sensors transportiert. Eine vom Prinzip ähnliche Vorgehensweise wird in US 6,143,164 beschrieben. Allerdings ist hier eine Blattfeder, die auf dem Sensor angeordnet ist und an einem Ende eine nadelförmige Spitze aufweist, auf die Sensoroberfläche aufzupressen, so dass die Lanzettenspitze dabei über das Sensorende hinaus in die Haut eindringt.

Insbesondere bei technischen Lösungen, die kein sprunghaftes oder sehr schnelles Lanzettieren der Haut erlauben, ist jedoch aufgrund der zu erwartenden psychologischen "Schmerzbarriere" seitens des Selbstanwenders mit einer zögerlichen Ausführung zu rechnen, so dass der Vorgang der Probegewinnung unsicher ausfällt oder wiederholt werden muss.

Für kombinierte Lanzetten-Sensorsysteme ist der Aufwand zur Fertigung der eher komplexen Einmalgebrauchs-Systeme vergleichsweise hoch. Sie bestehen häufig aus unterschiedlichen Materialien wie Kunststoffen und Metall, deren fachgerechte Entsorgung problematisch sein kann.

Aufgabe der Erfindung war es daher, ein Einmalgebrauchsmittel bereitzustellen, welches die genannten Nachteile nicht aufweist und insbesondere einfach, sicher und effektiv die Entnahme von Körperflüssigkeiten sowie der Detektion einzelner Parameter in dieser ermöglicht. Das Einmalgebrauchsmittel sollte weiterhin als Einzelelement manuell als auch in vormagazinierter Weise und durch eine gerätinterne Aktorik bedienbar sein.

Insbesondere sollte das Einmalgebrauchsmittel die Verletzungsgefahr durch einen ungewünschten Kontakt zwischen dem zur Lanzettierung befähigten Mittel mit der Haut minimal gehalten werden.

Die Erfindung löst diese Aufgabe durch Bereitstellung eines Einmalgebrauchsmittels umfassend einen Sensor und eine integrierte Lanzette für eine Lanzettierung der Haut zur Gewinnung einer Körperflüssigkeit, dadurch gekennzeichnet, dass die Lanzette einen kegelförmigen, formschlüssig angeordneten Dorn umfasst, der auf einer unter Druckbeaufschlagung sprunghaft und reversibel verformbaren Materialauswölbung positioniert ist und mindestens eine die Körperflüssigkeit aufnehmende, mit einer Reaktionsschicht des Sensors wirkverbindbare Nut und/oder einen Kanal aufweist.

Die anmeldungsgemäßen Einmalgebrauchsmittel weisen gegenüber den bisherigen zahlreichen Vorteile auf. Die Ausgestaltung der Materialauswölbung ist bisher in der Regel nicht so erfolgt, dass die Lanzette sprunghaft in das zu untersuchende Gewebe eindringen kann. Der Dorn der bekannten Mittel ist zwar unter Druckbeaufschlagung bewegbar, aber die Verformung insbesondere für den Einstichvorgang erfolgt nicht sprunghaft.

Für die sprunghafte Verformung ist ein Spannungsaufbau im Material in Kombination mit einer entsprechenden Materialflächenreserve zur Deformierung in die entgegengesetzte Richtung notwendig. Die Nadel bzw. der Dorn dringt daher bei den bekannten Mitteln proportional zur Kraft, die auf die Materialauswölbung einwirkt, in die Haut ein. Dies stellt einen entscheidenden Unterschied zur anmeldungsgemäßen Lehre dar, nach der der Einstich sprunghaft erfolgt, d.h. für den Anwender plötzlich und außerdem reversibel, da der Dorn auf einer unter Druckbeaufschlagung sprunghaft und reversibel verformbaren Materialauswölbung positioniert ist.

Das bekannte kraftproportionale Eindringen in die Haut stellt eine große psychologische Barriere für den Anwender dar, der auf Grund des zu erwartenden Schmerzes versuchen wird, entweder die Nadel langsamer und nicht so tief einzudrücken oder den aufliegenden, einzustechenden Finger etwas zurückzuziehen.

Durch diese Mängel der bekannten Mittel ist die Probengewinnung, d.h. die Bluttropfenbildung nicht optimal, wodurch das nachfolgende Testergebnis regelmäßig unsicher ausfällt.

Ein weiterer Unterschied zwischen dem erfindungsgemäßen Mittel und den im Stand der Technik offenbarten Einmalgebrauchsmittels ist das Vorhandensein einer wirkverbindbaren Nut und/oder Kanals und zwar in Kombination mit dem Merkmal des sprunghaften Eindringens. Diese Kombination führt zu zahlreichen überraschenden Resultaten. Durch die Nut ist eine bessere Ableitung der Probe möglich, denn diese kann, nachdem der Dorn wieder aus der Haut entfernt ist, vorteilhafterweise besser austreten. Durch die Verwendung einer Nut wird die effektive Kontaktoberfläche des Dorns zu den Nervenzellen (also nicht die Oberfläche des Dorns an sich) beim Eindringen in die Haut so gering wie möglich gehalten, wodurch das Schmerzempfinden im Vergleich zu den bekannten Lanzettenspitzen verringert wird. Überraschenderweise wird durch mindestens eine Nut zwar die Oberfläche des Dorns vergrößert, aber die effektive Kontaktoberfläche zu den Nervenzellen verringert. Bisherige Mittel weisen zum Teil eine Rinne auf einem planaren Fortsatz auf, in der z. B. die Blutgerinnung in Abhängigkeit von der Zeit bestimmt werden soll. Derartige Vorrichtungen in Form planar strukturierter Fortsätze an der Stirnseite eines Sensors bzw. eines Testsystems lösen das Problem der Schmerzverminderung nicht, völlig unabhängig davon, ob eine Blutgerinnungsrinne vorhanden ist oder nicht. Das Hautgewebe schmiegt sich in die Rinnen der planaren Fortsätze und damit liegen die Nervenzellen über die gesamte Oberfläche der Spitze an und bereiten dem Anwender Schmerzen. Die Vermeidungsstrategien des Anwenders ermöglichen dann keine genauen Ergebnisse.

Sofern gemäß anmeldungsgemäßer Lehre keine Nut aber ein Kanal im Dorn vorhanden ist, kann der Dorn auf Grund der effektiven Probenahme durch den Kanal besonders klein und daher schmerzmindernd ausgestaltet werden. Das Vorhandensein von Nut und Kanal ist für die Schmerzreduktion besonders vorteilhaft. Die Kombination aus den Merkmalen des sprunghaften Eindringens und der Nut/des Kanals führt zu einer Verbesserung und Verbilligung des Mittels sowie zu einer erhöhten Zuverlässigkeit, zur Beseitigung von Fehlern und zur Qualitätshebung.

Weiterhin ist zu berücksichtigen, dass die bekannten Mittel voraussetzen, dass nach dem Lanzettieren und dem Zurücknehmen des Dorns die Blutprobe unmittelbar und in ausreichender Menge austritt. Versuche, die seitens der Erfinder hierzu durchgeführt wurden, zeigen jedoch, dass dies üblicherweise nicht der Fall ist. Insbesondere bei stark vernarbten oder verhornten Fingerbeeren von Mehrfachnutzern von Einmalgebrauchsmitteln (z.B. Diabetikern) ist in der Regel ein Nachdrücken um die lanzettierte Stelle herum erforderlich, um für das Messmittel genügend Blut bzw. Probe austreten zu lassen.

Diese Problematik wird durch zahlreiche bekannte Vorrichtungen signifikant verstärkt, da nach dem Herausführen des Dorns das Messmittel auf der Probenahmestelle verbleibt und somit nicht sicher ist, ob genug Probe für die Messung zur Verfügung gestellt wird. Dieser entscheidende Umstand führt bei den bisherigen Mitteln zu Unsicherheiten in der Messung, weil bei jedem Sensor bzw. Messmittel eine Mindestmenge an Probe erforderlich ist, da anderenfalls die Messung fehlerhaft ausfällt.

Bei dem anmeldungsgemäßen Mittel ist durch die Kombination von (i) Dorn und Sensor, (ii) Nut und/oder Kanal und (iii) sprunghaftem Eindringen (= Verminderung der Schreck- und Meidreaktionen) eine Probenmenge von 0,1 µl bis 0,2 µl ausreichend, um eine sichere Bestimmung von einzelnen Parametern in der Probe zu ermöglichen. Das heißt, die Probenahme erfolgt durch Kombination gemäß den genannten Punkten (i) - (iii) dergestalt kontrolliert, dass erst, wenn die Messkammer des Sensors über den Innenkanal oder über die Nuten des Dorns befüllt wird, der Finger des Nutzers von der Probenahmestelle des Lanzettensensorsystems zu nehmen ist, wobei dieser Zeitpunkt beispielsweise durch ein Messgerät oder in einem Display signalisiert werden kann.

Die geringe Probemenge ist anmeldungsgemäß auch deshalb ausreichend, da das Einstechen für den Anwender plötzlich passiert, so dass Schreck- und Meidbewegungen das Detektieren der Probe auf dem Sensor nicht mehr nachteilig beeinflussen können. Weiterhin wird auf Grund der Kombination der Merkmale (i) Dorn und Sensor, (ii) Nut und/oder Kanal und (iii) sprunghaftem Eindringen eine Leistungssteigerung des Einmalgebruchsmittels bewirkt, so dass wegen der effektiveren Probengewinnung weniger Sensormaterial benötigt wird, was zu einer Verbilligung des Massenartikels Einmalgebrauchsmittel führt, da weniger kostenintensive Feinchemikalien eingesetzt werden müssen. Die Merkmalselemente beeinflussen und fördern sich gegenseitig und führen dadurch den Gesamterfolg (größere Effektivität, Leistungssteigerung, Verbilligung, erhöhte Zuverlässigkeit, Qualitätshebung) herbei, d.h. die Kombination der Merkmale (i) Dorn und Sensor, (ii) Nut und/oder Kanal und (iii) sprunghaftes Eindringen (d.h. die Positionierung des Dorn auf einer unter Druckbeaufschlagung sprunghaft und reversibel verformbaren Materialauswölbung) erschöpft sich nicht in einer Addition der Wirkung der Merkmalselemente.

Überraschenderweise ist es mit dem erfindungsgemäßen Einmalgebrauchsmittel möglich, Lanzette und Sensor so zu integrieren, dass ein Einwegsystem bereitgestellt wird, mit dem einfach, effektiv und insbesondere für den Anwender sicher eine Körperflüssigkeit, insbesondere Blut entnommen werden kann. Die Lanzettenspitze tritt nur während des Lanzettiervorgangs aus und verbleibt vor und nach der Lanzettierung unterhalb der Oberfläche des erfindungsgemäßen Einmalgebrauchsmittels, um die Verletzungsgefahr minimal zu halten. Die Lanzette ist hierbei insbesondere senkrecht auf einer Materialauswölbung positioniert, wodurch sie unter Druckbeaufschlagung sprunghaft heraustreten kann. Da die Materialauswölbung reversibel verformbar ausgestaltet ist, tritt die Lanzettenspitze nur während des gewünschten Vorganges aus dem Einmalgebrauchsmittel heraus. Durch das sprunghafte Heraustreten der Lanzettenspitze, die die Form eines Dorns aufweist und formschlüssig mit der reversibel verformbaren Materialauswölbung verbunden ist, wird die Haut durchdrungen. Die aus den verletzten Zellen austretende Körperflüssigkeit tritt in Kontakt mit der Oberfläche des Dorns und wird an einer oder mehreren Vertiefungen auf dem Dorn oder in einem Kanal im Dorn auf Grund von Kapillarkraftwirkung abgeleitet und dem Einmalgebrauchsmittel, das mit der Lanzettierspitze wirkverbindbar ist, zugeführt. Hierdurch wird ein Kontakt zwischen der entnommenen Probe und der Reaktionsschicht des Sensors hergestellt.

Je nach Ausgestaltung der Reaktionsschicht des Sensors können so einzelne Parameter der entnommenen Körperflüssigkeit, beispielsweise Blut, detektiert werden. Bei diesen einzelnen Parametern kann es sich beispielsweise um den Glucose- oder den Laktatwert des Blutes handeln.

Nach einer bevorzugten Ausführungsform der Erfindung ist der Dorn symmetrisch oder zentrisch positioniert. Vorteilhafterweise ist hierdurch gewährleistet, dass der Dorn während der Lanzettierung schnell und sicher in das gewünschte Hautareal eindringt.

In einer weiteren bevorzugten Ausführungsform der Erfindung weist die Außenwandung des Dorns mindestens eine sich in ihrer Tiefe vergrößernde Nut 6 und/oder im Inneren von der Spitze bis in den unteren Bereich führenden Kanal 7 auf, der im unteren Bereich an einer Stelle der Außenwandung austritt, wobei das Ende der Nut 8a und/oder die Austrittsöffnung 8b des Kanals 7 mit der kapillaren Eintrittsöffnung 11a des Sensors 11 über eine formschlüssige Verbindung wirkverbindbar ist.

In einer weiteren bevorzugten Ausführungsform der Erfindung besteht die Lanzette aus einem kegelförmigen Dorn 1 und einer Fußfläche 3 mit einer oder mehreren unter mechanischem Druck sprunghaft und reversibel verformbaren Materialauswölbung(en) 3a, 3c, d, f auf dem der kegelförmige Dorn 1 formschlüssig, symmetrisch oder zentrisch positioniert ist, dessen Außenwandung von der Spitze 2 bis in den unteren Bereich eine oder mehrere sich in ihrer Tiefe vergrößernde Nuten 6 oder im Inneren von der Spitze bis in den unteren Bereich einen inneren Kanal 7 aufweist, der im unteren Bereich an einer Stelle der Außenwandung austritt und bei dem das Ende der Nut 8a oder die Austrittsöffnung 8b des inneren Kanals 7 mit der kapillaren Eintrittsöffnung 11a des Einmalgebrauchssensors 11 über eine formschlüssige Verbindung zu einem Trägersubstrat 12 während des Lanzettiervorgangs durch eine sprunghafte, reversible Verformung der Materialauswölbungen 3a, 3c, d, f der Fußfläche 3, die durch mechanischen Druck ausgelöst wird, in Kontakt gebracht werden.

An der Außenwandung des kegelförmigen Dorns 1 sind von der Spitze bis in den unteren Bereich eine oder mehrere Nuten 6 angeordnet oder im Inneren von der Spitze bis in den mittleren Bereich ein Kanal 7, der im unteren Bereich an einer Stelle der Außenwandung austritt. Das Ende einer der Nuten 8a oder die Austrittsöffnung des inneren Kanals 8b wird mit der kapillaren Eintrittsöffnung 11a des Einmalgebrauchssensors 11 während des Lanzettiervorgangs durch eine reversible sprunghafte Verformung der Materialauswölbung der Fußfläche 3, die durch mechanischen Druck ausgelöst wird, in Kontakt gebracht.

Dem Fachmann ist bekannt, dass Einmalgebrauchsmittel, die einen Sensor umfassen, häufig auch als Einmalgebrauchssensor bezeichnet werden, wobei in diesem Fall die Formulierung Einmalgebrauchssensor weniger den bestimmten Sensor betrifft, als das gesamte Gebrauchsmittel. Demgemäß werden im Sinne der Erfindung Einmalgebrauchssensor und Einmalgebrauchsmittel im Wesentlichen im gleichen Sinne verwendet.

Bei einem derartigem Einmalgebrauchsmittel oder Einmalgebrauchssensor mit integrierter Lanzette werden zunächst der Sensor und die Lanzette separat gefertigt und im letzten Schritt in planarer Form kraft- und formschlüssig durch ein Trägersubstrat miteinander verbunden. Als Einmalgebrauchssensor 11 dient beispielsweise ein Sensoraufbau wie in der Patentanmeldung P/102 34 564.3 beschrieben.

Die Lanzette des erfindungsgemäßen Einmalgebrauchssensors mit integrierter Lanzette verwendet als Lanzettenspitze einem kegelförmigen Dorn 1, dessen Höhe zwischen 1 mm und 3 mm beträgt. Der Durchmesser der Spitze 2 des Dorns 1 beträgt zwischen 5 µm und 30 µm und der Durchmesser an der Fußfläche 3 des Dorns zwischen 1,5 mm und 3 mm. Der kegelförmige Dorn 1 weist im unteren Drittel einen zylinderförmigen Abschnitt 4 auf. Der zylinderförmige Abschnitt 4 mit einem Durchmesser zwischen 0,2 mm und 0,4 mm ist zwischen 0,1 mm und 0,4 mm hoch. Dadurch, dass der zylinderförmige Abschnitt 4 treppenförmig in den kegelförmigen Dorn 1 eingeformt ist, wird am Ende des zylinderförmigen Abschnitts 4 ein kleiner Absatz 5 zwischen 0,1 mm und 0,2 mm ausgebildet. Der aus Kunststoff mittels Heißprägen, Mikrospritzgusstechnik oder im Tiefziehverfahren und in Kombination mit Laserstrukturierung hergestellte kegelförmige Dorn 1 ist massiv und mit seiner Fußfläche 3 kraft- und formschlüssig verbunden.
(a) Eine weitere Ausführungsform der sprunghaft und reversibel verformbaren Fußfläche 3 wird erhalten, wenn in eine rechteckige dünne und plastische Folie mit einer Breite zwischen 4 mm und 10 mm, einer Länge zwischen 10 mm bis 30 mm und mit einer Dicke zwischen 0,1 mm und 0,5 mm entlang der symmetrischen Längsachse eine runde wannenförmige Auswölbung 3a eingebracht wird, die nach unten eine Tiefe zwischen 1 mm und 2 mm und eine Breite zwischen 3 mm und 7 mm aufweist. In der Auswölbung ist im ersten Drittel zentrisch und nach oben weisend der kegelförmige Dorn 1 angeordnet. Dabei werden die Fußflächenränder links und rechts bis zur virtuellen Querachse des kegelförmigen Dorns 1 durch Führungsschienen 12a,b des Trägersubstrats 12 oben und unten straff geführt und fixiert. Eine zusätzliche Querstrebe 12c verläuft entlang der Querachse des kegelförmigen Dorns 1 und tangiert dort die tiefste Stelle der runden Auswölbung. Der verbleibende Rest der plastischen Folie ist freitragend, wobei eine bestimmte Fläche 3b über das Ende des Trägersubstrates hinaus ragt. Bei mechanischem Druck von oben auf die freiliegende rechteckige Fläche 3b verbiegt sich die Rechteckfläche bis zur Querstrebe 12c bzw. bis zum Beginn der geführten Ränder in den Führungsschienen 12a,b, die gleichzeitig eine Gegendruckfläche bilden. Dadurch weicht die nach unten ausgeformte Auswölbung 3a an der Stelle der Querstrebe 12c sprunghaft nach oben aus, so dass der zentrisch auf der Auswölbung angeordnete kegelförmige Dorn 1 sprunghaft nach oben bewegt wird. Wenn der Druck von der freiliegenden rechteckigen Fläche genommen wird, springt die runde wanneförmige Auswölbung 3a zurück in die Ausgangslage.
(b) Eine weitere Ausführungsform der sprunghaft und reversibel verformbaren Fußfläche 3 wird erhalten, wenn die Fußfläche 3 des kegelförmigen Dorns eine oder mehrere sprunghaft und reversibel verformbare, im Querschnitt kreiswellenförmig geschlossene oder segmentartig ausgeformte Auswölbungen (3c,d) nach oben und unten aufweist und mit einer plastisch verformbaren Kunststofffolie 10, die entgegen der Kegelspitze ausgewölbt ist, mit dem ebenen, rechteckigen Rand der Fußfläche 3 form- und kraftschlüssig verbunden wird.

Das Verhältnis der nach oben und unten ausgeformten Auswölbungen 3c,d der Fußfläche 3 ist derart, dass bei Ausübung eines sprunghaften mechanischen Druckes beispielsweise durch Eindrücken der Auswölbung der plastisch verformbaren Kunststofffolie 10 diese auf die unterste Auswölbung 3f des Fußes 3 trifft, die über die Auswölbungen 3c,d nach oben ausweicht, wobei der kegelförmige Dorn 1 auch sprunghaft nach oben bewegt wird. Beim Nachlassen des mechanischen Drucks auf die plastisch verformbare Kunststofffolie 10 springt diese in ihre Ausgangsposition zurück und auch die kreiswellenförmig geschlossenen oder segmentartig ausgeformten Auswölbungen 3c,d der Fußfläche 3 nehmen wieder ihre ursprüngliche Lage ein, so dass die unterste Auswölbung, auf deren Innenseite der kegelförmige Dorn positioniert ist, ebenfalls in die Ausgangsposition zurückkehrt.

Diese reversible Entspannungsvorrichtung allerdings ohne die plastisch verformbare Kunststofffolie 10 ist besonders für eine magazinierte Integration in eine Vorrichtung, die mittels einer von der Vorrichtung kontrollierten Aktorik betätigt wird, geeignet.

Von der Spitze 2 des kegelförmigen Dorns verlaufen bis zum unteren Teil eine oder mehrere schmale Nuten 6 mit einer Breite und Tiefe zwischen 20 µm und 180 µm, die an der Kante 9 enden, an der die Kegelform in einen zylinderförmigen Abschnitt übergeht. Die Tiefe der Nut oder Nuten vergrößert sich von der Spitze zum unteren Teil des Dorns. Je mehr Nuten angeordnet sind, desto schneller wird die Gewinnung von Blut oder Gewebsflüssigkeit erfolgen. Ein weiterer Vorteil der Nuten besteht darin, dass die Außenfläche des kegelförmigen Dorns, die während der Lanzettierung Kontakt mit Nervenzellen erhalten geringer und damit das Schmerzempfinden des Selbstanwenders vermindert wird.

In einer anderen Ausführungsform führt ein Kanal 7 im Inneren des massiven Dornenteils von der Spitze des kegelförmigen Dorns nach unten und tritt unmittelbar über der Kante 9, an der der Dorn einen zylinderförmigen Abschnitt 4 aufweist, aus. Das untere Ende der Nut 8a auf der Außenfläche des Kegels bzw. die Austrittsöffnung des Kanals 8b wird genau symmetrisch gegenüber der Stirnseite des Sensors 11, an dem sich die Probeeintrittsöffnung 11a des Sensors 11 befindet, angeordnet. Der kegelförmige Dorn 1 und der Sensor 11 werden räumlich derart zueinander angeordnet, dass bei der sprunghaften und reversiblen Verformung einer Materialauswölbung oder mehrerer Materialauswölbungen der Fußfläche 3 die vertikale Bewegung des kegelförmigen Dorns 1 aufgrund des Absatzes 5 unterhalb der Fläche an der Stirnseite des Sensors 1 begrenzt wird. Auf diese Weise werden das Ende der Nut 8a bzw. die Austrittsöffnung des Kanals 8b reproduzierbar auf die gleiche Höhe gebracht und tangieren sich. Gegebenfalls enthält die Probeeintrittsöffnung 11a des Sensors 11 einen Docht zur erleichterten Probeübernahme.

Die Positionierung und formschlüssige Aufnahme von Lanzettiereinheit und Sensorelement erfolgt mittels eines in Längsrichtung stufenförmig ausgesparten Trägersubstrats 12. Das Trägersubstrat 12 ist analog dem Sensorelement zwischen 4 mm und 10 mm breit und zwischen 15 mm und 60 mm lang. Die Höhe des Trägersubstrates 12 beträgt zwischen 2 mm und 4 mm.

An dem Ende des Trägersubstrates 12, an dem die Lanzette und der Sensor 11 mit der Probeeintrittsöffnung 11a gegenüberstehend angeordnet werden, weist das Trägersubstrat 12 zwei von oben nach unten ineinander übergehende, durchgehende runde Aussparungen auf. Die auf der Oberseite des Trägersubstrates 12 befindliche Vertiefung 13 ist teilkreisförmig mit einem Durchmesser zwischen 0,1 mm und 0,8 mm und einer Höhe zwischen 0,1 mm und 0,5 mm und die von der Unterseite des Trägersubstrates 12 aus beginnende untere Vertiefung 14 ist kegelstumpfförmig mit einem Basisdurchmesser zwischen 3 mm und 8 mm, einen Spitzendurchmesser zwischen 0,1 mm und 0,8 mm und einer Höhe zwischen 0,5 mm und 2,5 mm ausgebildet.

In die Aussparungen wird der kegelförmige Dorn 1 aufgenommen und über die Ränder seiner Fußflächen 3, die in die entsprechenden Aufnahmeschienen links und rechts 12a,b an den Rändern des Trägersubstrats 12 bis zur Position der virtuellen Querachse des kegelförmigen Dorns 1 geführt werden, mittels Klebetechnik oder Ultraschallschweißen mit dem Trägersubstrat punktuell kraft- und formschlüssig verbunden.

Alle beschriebenen Teile werden aus Kunststoffen mittels Mikrospritzguss, Heißprägetechnik oder im Tiefziehverfahren und in Kombination mit Laserablation gefertigt. Als Material sind Folien oder Platten aus Polycarbonat, Polymethylmethacrylat, Polystyrol, Polyether, Polyethylenterephthalat, Acrylnitril-Butadien-Styrol-Copolymer, Polyvinylchlorid, thermoplastisches Elastomere oder Kombinationen davon mit Dicken zwischen 0,1 mm und 4,0 mm geeignet.

Für die Herstellung der Fußfläche 3 mit einer oder mehreren sprunghaft und reversibel verformbaren kreiswellenförmig geschlossenen oder segmentartig ausgeformten Auswölbungen 3c,d nach oben und unten wird vorzugsweise ein Zweikomponentenspritzgussverfahren genutzt, wobei für den Abguss dieser Auswölbungen 3c,d ein thermoplastisches Elastomer verwendet wird.

Die Erfindung betrifft auch die Verwendung des Einmalgebrauchsmittels für die Lanzettierung der bzw. durch die Haut. Es ist in einer bevorzugten Ausführungsform der Erfindung vorgesehen, dass hierbei eine Körperflüssigkeit durch die Haut entnommen wird. Die Entnahme der Körperflüssigkeit kann beispielsweise insbesondere erfolgen, um aus dieser die Glucose- und/oder Laktatwerte zu bestimmen.

Das erfindungsgemäße Einmalgebrauchssensor mit integrierter Lanzette wird wie folgt angewandt:

Unter Verwendung der ersten Ausführungsform gemäß (a) legt der Nutzer für die manuelle Lanzettierung den Finger, von dem die Probenahme erfolgen soll, fest in die obere Aussparung 13 des Trägersubstrates 12 und drückt mit einem zweiten Finger die verlängerte rechteckige Fußfläche 3b nach unten, so dass aufgrund der Verformung dieser Fläche bis zur Querstrebe 12c, die entlang der Querachse des darüber angeordneten kegelförmigen Dorns 1 angeordnet ist und als Auflagepunkt für die Auswölbung 3a maßgeblich deren Ausweichen nach oben auslöst, der darauf befindliche kegelförmige Dorn 1 sprunghaft nach oben bewegt wird.

Bei der manuellen Lanzettierung gemäß (b) legt der Nutzer den Finger, an dem die Probenahme erfolgen soll, fest in die obere Aussparung 13 des Trägersubstrates 12 und drückt mit dem Daumen der gleichen Hand von der Unterseite des Trägersubstrates 12 zentrisch so auf die Auswölbung der geprägten Kunststofffolie 10, dass diese auf die unterste Auswölbung 3f des Fußes 3 trifft, die über die Auswölbungen 3c,d ebenfalls sprunghaft nach oben ausweicht, wobei der kegelförmige Dorn 1 sprunghaft nach oben bewegt wird.

In beiden Ausführungsformen erfolgt eine vertikale Bewegung des kegelförmigen Dorns 1 nach oben. Er stößt dabei mit dem Absatz 5 des zylinderförmigen Abschnitts an die Unterseite der Stirnfläche des Sensors 11, so dass gleichzeitig das Ende der Nut 8a auf der Oberfläche des Dorns 1 bzw. die Austrittsöffnung des Kanals 8b die kapillare Eintrittsöffnung 11a des Sensors tangiert. Durch die sprunghafte vertikale Bewegung nach oben dringt die Spitze 2 des kegelförmigen Dorns in die Haut ein und verletzt bei entsprechender Eindringtiefe kapillarbluttransportierende Zellen. Die Nut oder Nuten 6 auf der Außenfläche des Dorns bzw. der Kanal 7 im Inneren des Dorns 1 transportiert aufgrund der Kapillarkraftwirkung interstitielle Flüssigkeit oder Kapillarblut bis zum Ende der Nut 8a bzw. bis zur Austrittsöffnung 8b des Kanals, die an dieser Stelle direkt weiter über die kapillare Eintrittsöffnung 11a des Sensors 11 oder über einen Docht in die Messkammer 11b des Sensors 11 geleitet und dort detektiert wird. Die zur Detektion erforderliche Probemenge beträgt zwischen 0,1 µl und 0,5 µl Probe.

Nach der Probenahme, die über das Stromansprechverhalten des Sensors 11 kontrolliert und akustisch bestätigt wird, nimmt der Anwender Finger und Daumen vom Einmalgebrauchssensor mit integrierter Lanzette.

Bei der ersten Ausführungsform entspannt sich die Fußfläche 3 und die runde Auswölbung 3a kehrt in ihre Ausgangsform nach unten zurück, wobei der kegelförmige Dorn wieder innerhalb des Trägersubstrates 12 versenkt wird.
Bei der zweiten Ausführungsform gelangt in analoger Weise die Auswölbung der geprägten Kunststofffolie 10 bei Nachlassen des mechanischen Drucks in ihre Ausgangsstellung zurück und auch die im Querschnitt kreiswellenförmigen Auswölbungen 3c,d der Fußfläche 3 kehren aufgrund ihrer vorgeprägten Materialausformung und ihrer elastischen Materialeigenschaften in ihre Ausgangspositionen zurück, so dass die Spitze 2 des kegelförmigen Dorns 1 wieder innerhalb des Trägersubstrates versenkt wird.

Damit wird in beiden Ausführungsformen nach der Verwerfung des erfindungsgemäßen Einmalgebrauchssensor mit integrierter Lanzette gesichert, dass keine Verletzungsgefahr durch die mit Blut kontaminierte Spitze 2 gegen Dritte ausgeht.

Der erfindungsgemäße Einmalgebrauchssensor bzw. das Einmalgebrauchsmittel mit integrierter Lanzette kann auch Bestandteil einer Vorrichtung sein, in deren Gehäuse 15 gleichzeitig die Hard- und Software für die Signalverarbeitung und Messwertanzeige integriert ist. Bevorzugt zur Anwendung kommt dabei die zweite Ausführungsform des erfindungsgemäßen Einmalgebrauchssensors mit integrierter Lanzette, die als Fußfläche 3 eine oder mehrere sprunghaft und reversibel verformbare im Querschnitt kreiswellenförmig geschlossene oder segmentartig ausgeformte Auswölbungen nach oben und unten aufweist aber auf die Kunststofffolie 10 mit einer geprägten Auswölbung verzichtet.

In dieser Anwendungsform wird der Einmalgebrauchssensor mit integrierter Lanzette beispielsweise in einem Stapelmagazin 16 bevorratet und in der Vorrichtung durch einen geeigneten Federmechanismus 17 und einen ersten Aktor 18 für die Messung bereitgestellt. Während der Bereitstellung schiebt der Aktor 18 den Einmalgebrauchssensor mit integrierter Lanzette derart in ein nach außen offenes Applikationsfenster 20, dass die obere Aussparung 13 des Sensorsubstrates für den Anwender zugänglich wird. Gleichzeitig erfolgt eine elektrische Kontaktierung des Sensors 11 zum Potentiostaten der Vorrichtung 15.

Beim Andrücken des Fingers, an dem die Probenahme erfolgen soll, in die obere Aussparung 13 des Trägersubstrates 12 wird gleichzeitig von der Unterseite eine sprunghafte mechanische Bewegung durch den Aktor 19 zentrisch auf die unterste Auswölbung 3f der Fußfläche ausgeübt, so dass die im Querschnitt kreiswellenförmigen Auswölbungen 3c,d der Fußfläche 3 in Richtung Dornspitze 2 sprunghaft verformt werden. Dadurch erfolgt eine vertikale Bewegung des kegelförmigen Dorns 1 und stößt dabei mit dem Absatz 5 des zylinderförmigen Abschnitts an die Unterseite der Stirnfläche des Sensors 1, so dass gleichzeitig das Ende der Nut 8a auf der Oberfläche des Dorns 1 bzw. die Austrittsöffnung des Kanals 8b die kapillare Eintrittsöffnung 11a des Sensors tangiert. Durch die sprunghafte vertikale Bewegung nach oben dringt die Spitze 2 des kegelförmigen Dorns 1 in die Haut ein und verletzt bei entsprechender Eindringtiefe kapillarbluttransportierende Zellen. Die Nut oder die Nuten 6 auf der Außenfläche des Dorns 1 transportieren aufgrund der Kapillarkraftwirkung interstitielle Flüssigkeit oder Kapillarblut bis zum unteren Ende der Nut oder Nuten 8a des Kanals. Die interstitielle Flüssigkeit oder das Kapillarblut werden an dieser Stelle direkt weiter über die kapillare Eintrittsöffnung 11a des Sensors 11 oder über einen Docht in die Messkammer 11b des Sensors 11 geleitet und dort detektiert. Die zur Detektion erforderliche Probemenge beträgt zwischen 0,1 µl und 0,5 µl. Nach der Probenahme, die über das Stromansprechverhalten des Sensors 11 kontrolliert und akustisch bestätigt wird, nimmt der Anwender den Finger vom Einmalgebrauchssensor mit integrierter Lanzette und der Aktor 19 der Vorrichtung wird zurückgezogen. Dadurch gelangen die die im Querschnitt kreiswellenförmigen Auswölbungen 3c,d der Fußfläche 3 aufgrund ihrer vorgeprägten Materialausformung und elastischen Materialeigenschaften in ihre Ausgangspositionen zurück, so dass die Spitze des kegelförmigen Dorns 2 wieder innerhalb des Trägersubstrates versenkt wird. Der Sensor wird in ein internes Abfallmagazin 21 der Vorrichtung ausgeworfen.

Schließlich kann der erfindungsgemäße Einmalgebrauchssensor mit integrierter Lanzette auch zu einer separaten Probenahme und anschließenden Ausmessung benutzt werden. In dieser Anwendungsform entfernt der Nutzer unmittelbar nach der Lanzettierung, die wie bereits durch die vorangegangenen Anwendungsformen beschrieben erfolgt, den Finger von der Ausformung des Trägersubstrates und wartet solange, bis an der lanzettierten Hautstelle sich ein Bluttropfen gebildet hat. Die Fingerspitze wird danach so in die obere Aussparung 13 des Trägersubstrates 12 angelegt, dass der Bluttropfen von der kapillaren Probeeingangsöffnung 11a des Sensors aufgenommen wird und zur Detektion gelangt.

Die Erfindung betrifft auch ein Verfahren zur Herstellung des erfindungsgemäßen Einmalgebrauchsmittels, wobei der Sensor und die Lanzette separat gefertigt werden und nachfolgend in planarer Form kraft- und formschlüssig durch ein Trägersubstrat miteinander verbunden werden.

Bevorzugt ist es in einer Ausführungsform der Erfindung, dass die Einzelbestandteile des Sensors und der Lanzette aus Kunststoffen mittels Mikrospritzguss, Heißprägetechnik und/oder Tiefziehverfahren hergestellt werden, wobei in einer besonders bevorzugten Ausführungsform der Kunststoff Polycarbonat, Polymethylacrylat, Polysterol, Polyether, Polyethylenterephterphtalat, Acrylnitril-Butadien-Styrol-Copolymer, Polyvinylchlorid, thermoplastische Elastomere und/oder Kombinationen aus diesen umfasst.

### Ausführungsbeispiele

Der erfindungsgemäße Einmalgebrauchssensors mit integrierter Lanzette wird durch nachfolgende Ausführungsbeispiele und Zeichnungen näher erläutert.

### Beispiel 1:

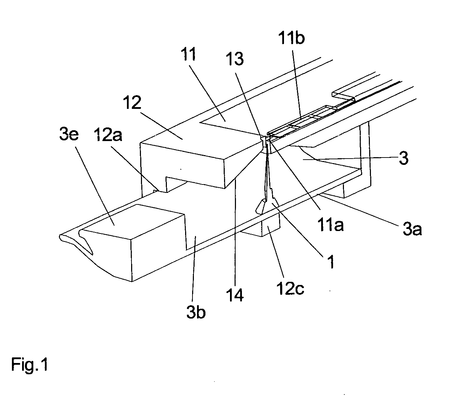

Erfindungsgemäßer Einmalgebrauchssensors mit integrierter Lanzette zum Nachweis von Glucose. Zur Erläuterung dienen Fig. 1 bis Fig. 3.

Der erfindungsgemäße Einmalgebrauchssensor mit integrierter Lanzette besteht im Einzelnen aus einer Lanzette, einem Sensor und einem Trägersubstrat, die zunächst separat voneinander hergestellt und im Anschluss formschlüssig zusammengefügt werden.

Die Lanzette des erfindungsgemäßen Einmalgebrauchssensors mit integrierter Lanzette wird mittels Mikrospritzgussverfahren wie folgt strukturiert: Aus Polycarbonat wird mit den Abmaßen 15 mm x 7 mm (LxB) und einer Stärke von 0,2 mm eine Fußfläche 3 mit einer wannenähnlichen runden Auswölbung 3a entlang der Symmetrielängsachse erzeugt, die an ihrer tiefsten Stelle 0,9 mm nach unten ausgewölbt ist. Im vorderen Drittel wird zentrisch in die Auswölbung ein massiver, nach oben weisender kegelförmiger Dorn 1 angespritzt. Der kegelförmige Dorn ist 2,2 mm hoch. Der Durchmesser der Spitze 2 des Dorns 1 beträgt 5 µm und der Durchmesser auf der Fußfläche 3 des Dorns beträgt 1 mm. Der kegelförmige Dorn 1 weist in einem Abstand von 0,35 mm von der Fußfläche einen annähernd zylinderförmigen Abschnitt 4 auf. Der kegelförmige Abschnitt 4 mit einem Durchmesser von 0,3 mm ist 0,35 mm lang. Dadurch, dass der zylinderförmige Abschnitt 4 treppenförmig in den kegelförmigen Dorn 1 eingeformt ist, wird am Ende des zylinderförmigen Abschnitts 4 ein kleiner Absatz 5 von 0,1 mm ausgebildet. Vom Ende des zylinderförmigen Abschnitts des Dorns verjüngt sich der Dorn über eine Höhe von 1,5 mm bis zur Spitze. Von der Spitze des Kegels verläuft bis zur Kante 9, an der die Kegelspitze in einen zylinderförmigen Abschnitt 4 übergeht, eine schmale Nut 6 mit einem Durchmesser von 3,0 µm an der Spitze und 120 µm an der Kante 9, die nach außen derart versetzt ist, dass an der Spitze 2 des Dorns noch eine Materialkante von ca. 5 µm stehenbleibt und an der Kante die tiefste Stelle der Nut 90 µm beträgt.

Am gegenüberliegenden Ende der rechteckigen Grundfläche ist auf einer Fläche von 6 mm x 3 mm (BxL) eine quaderförmige Druckauflagefläche 3e von 2,4 mm angegossen.

Der amperometrische Glucosesensor für den Einmalgebrauch wird wie in Beispiel 1 der Schutzanmeldung P/102 34 564.3 beschrieben, hergestellt. Der Sensor hat Außenabmessungen von 6 mm x 35 mm x 0,6 mm (BxLxH) und weist an seiner Stirnfläche eine kapillare Probeaufnahmeöffnung von 0,2 mm x 0,1 mm (BxH) auf. Die Messkammer des Sensors mit einem Volumen von 0,3 µl enthält neben einer potentiostatischen Dreielektrodenanordnung ein Glucoseoxidase-Mediator-Gemisch einschließlich Additiven für eine schnelle Benetzbarkeit der Reagenzien. Am entgegengesetzten Ende des Sensors befinden sich Kohlenstoffkontaktbahnen zum Auslesen des Messsignals mittels eines Handmessgerätes.

Zur formschlüssigen Integration von Lanzette und Sensor wird ein Trägersubstrat verwendet.

Das Trägersubstrat mit Außenmaßen von 7 mm x 21,4 mm x 3,1 mm (LxBxH) ist stufenartig ausgespart und nimmt von einem Ende die Lanzette und von dem anderen Ende den Sensor derart auf, dass sich die Lanzettenspitze und die Probeeintrittsöffnung während des Lanzettiervorgangs tangieren. Der Sensor wird in eine vorgesehene Vertiefung des Trägersubstrats verklebt, so dass die Sensoroberfläche und die Oberfläche des Trägersubstrats zueinander eben sind. Darüber hinaus weist dieses Ende von oben nach unten ineinander übergehende und durchgehende Vertiefungen zur Aufnahme des kegelförmigen Dorns 1 von unten auf. Die obere Vertiefung 13 ist teilkreisförmig mit einem Durchmesser von 0,5 mm und einer Höhe von 0,3 mm und die untere Vertiefung 14 ist kegelstumpfförmig mit einem Basisdurchmesser von 4,9 mm, einen Spitzendurchmesser von 0,5 mm und einer Höhe von 0,7 mm ausgebildet.

Die Fußfläche 3 des kegelförmigen Dorns 1 wird an seinen Rändern in Führungsschienen 12a,b, die links und rechts an dem Trägersubstrat angeordnet sind, bis zur virtuellen Querachse des kegelförmigen Dorns 1 oben und unten geführt und punktuell durch Ultraschallschweißen fixiert. Eine zusätzliche Querstrebe 12c verläuft entlang der Querachse des kegelförmigen Dorns 1 und tangiert dort die tiefste Stelle der runden Auswölbung. Der verbleibende Rest der plastischen Folie ist freitragend, wobei die Fläche 3b mit der angegossenen quaderförmigen Druckauflagefläche 3e über das abgestufte Ende des Trägersubstrates 12 hinaus ragt.

Bei mechanischem Druck von oben auf die quaderförmige Andruckfläche 3e verbiegt sich die Rechteckfläche bis zu den geführten Rändern bzw. bis zur Querstrebe 12c, die gleichzeitig eine Gegendruckfläche bilden. Mit der Materialverformung wird eine mechanische Spannung im Material erzeugt, die an der Gegendruckstelle der Querstrebe 12c, dazu führt, dass das Material bzw. die nach unten ausgeformte Auswölbung 3a sprunghaft nach oben ausweicht. Der zentrisch auf der Auswölbung angeordnete kegelförmige Dorn 1 wird dabei sprunghaft nach oben bewegt. Wenn der Druck von der freiliegenden rechteckigen Fläche genommen wird, springt die kreisförmige Auswölbung 3a zurück in die Ausgangslage.

Nach der Kontaktierung dieses Glucoseeinmalgebrauchssensors mit integrierter Lanzette mit einem potentiostatischen Messgerät, das für die geeignete Polarisationsspannung, Stromverstärkung, Messablaufsteuerung und Messwertanzeige sorgt, erfolgt die Probenahme und Ausmessung wie folgt: Der Nutzer legt den Finger, von dem die Blutprobe entnommen werden soll, fest in die obere Aussparung 13 des Trägersubstrates 12 bei gleichzeitigem Gegendruck durch den Daumen der gleichen Hand von der Unterseite des Trägersubstrats. Mit einem benachbarten Finger drückt er die quaderförmige Druckauflagefläche 3e am Rand der freitragenden überstehenden Fläche 3b der Fußfläche 3 nach unten, so dass aufgrund der Verformung dieser Fläche auch die runde nach unten weisende Auswölbung 3a über der Querstrebe 3b entlang der virtuellen Querachse des kegelförmigen Dorns 1 nach oben ausweicht und damit der darauf befindliche kegelförmige Dorn 1 sprunghaft nach oben bewegt wird und dabei die Haut penetriert. Bei dieser vertikalen Bewegung stößt der kegelförmige Dorn 1 mit dem Absatz 5 des zylinderförmigen Abschnitts 4 an die Unterseite der Stirnfläche des Sensors 11 an, so dass gleichzeitig das Ende der Nut 8a auf der Oberfläche des kegelförmigen Dorns 1 die kapillare Probeeintrittsöffnung 11a des Sensors tangiert.

Bei entsprechender Eindringtiefe der Spitze 2 des kegelförmigen Dorns 1 in die Haut werden kapillarbluttransportierende Zellen verletzt. Das austretende Kapillarblut wird aufgrund der Kapillarkraftwirkung in der Nut 6 auf der Außenfläche des Dorns bis zum Ende der Nut 8a transportiert und sammelt sich dort an. Dadurch kann die Blutprobe von der kapillaren Probeeintrittsöffnung des Sensors übernommen und in die Messkammer des Sensors geleitet werden. Die zur Detektion erforderliche Probemenge beträgt zwischen 0,1 µl und 0,5 µl. Sobald die Messkammer gefüllt ist wird ein Triggerstrom erzeugt, der durch ein akustisches Signal dem Nutzer signalisiert, dass die Probenahme, die ca. 2 sec bis 5 sec dauert, beendet ist.

Wenn dann der Anwender Finger und Daumen von dem Glucoseeinmalgebrauchssensor mit integrierter Lanzette nimmt, entspannt sich die Fußfläche 3 und die runde Auswölbung 3a kehrt in ihre Ausgangsform nach unten zurück, wobei der kegelförmige Dorn 1 wieder innerhalb des Trägersubstrates 12 versenkt wird.

Dadurch wird nach der Verwerfung des erfindungsgemäßen Einmalgebrauchssensors mit integrierter Lanzette gesichert, dass keine Verletzungsgefahr durch die mit Blut kontaminierte Spitze 2 gegen Dritte ausgeht.

### Beispiel 2

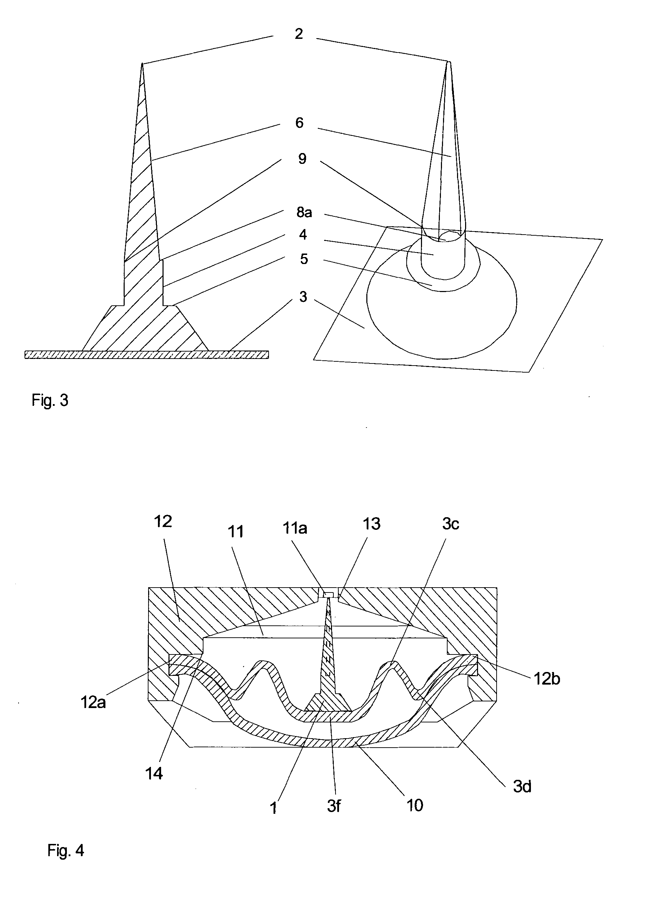

Erfindungsgemäßer Einmalgebrauchssensor mit integrierter Lanzette zum Nachweis von Glucose. Zur Erläuterung dienen Fig. 3 und Fig. 4.

Der erfindungsgemäße Einmalgebrauchssensor mit integrierter Lanzette besteht im Einzelnen aus einer Lanzette, einem Sensor und einem Trägersubstrat, die zunächst separat voneinander hergestellt und im Anschluss formschlüssig integriert werden.

Die Lanzette des erfindungsgemäßen Einmalgebrauchssensors mit integrierter Lanzette wird mittels eines Zweikomponenten-Mikrospritzgussverfahren wie folgt strukturiert: Aus Polycarbonat wird ein kegelförmiger Dorn 1 mit einer Gesamthöhe von 2,2 mm gegossen. Die Form und die Abmaße des kegelförmigen Dorns entsprechen denen wie in Beispiel 1 beschrieben und die Strukturierung ist in Abb. 3 dargestellt. An den kegelförmigen Dorn 1 wird eine Fußfläche 3 aus einem thermoplastischen Elastomer angegossen. Die Fußfläche weist jeweils eine sprunghaft und reversibel verformbare, im Querschnitt kreiswellenförmig ausgeformte geschlossene Auswölbung 3c,d nach oben und unten auf. Die Folienstärke der Fußfläche 3 mit den Auswölbungen 3c,d beträgt im Mittel 0,2 mm. Die Durchmesser des inneren, mittleren und äußeren Wölbungskreises sind 0,6 mm, 1,3 mm und 1,9 mm. Die Höhe der nach oben weisenden Auswölbung ist 1 mm, die Höhe der nach unten weisenden Wölbung beträgt 0,8 mm vom höchsten Punkt der nach oben weisenden Auswölbung.

Eine plastisch verformbare Polykarbonatfolie 10 mit einer Grundfläche von 7 mm x 7 mm und einer Materialdicke von 0,2 mm, die kreisförmig mit einem Durchmesser von 4 mm und einer Tiefe von 1,5 mm ausgewölbt ist, wird mit der Ausformung nach unten an den Rändern mit der Unterseite der Fußfläche mittels Ultraschallschweißen verbunden.

Der amperometrische Glucosesensor für den Einmalgebrauch wird wie in Beispiel 1 hergestellt.

Zur formschlüssigen Integration von Lanzette und Sensor wird ein Trägersubstrat verwendet.

Das Trägersubstrat mit Außenmaßen von 30 mm x 7 mm x 3,1 mm (LxBxH) ist stufenartig ausgespart und nimmt von einem Ende die Lanzette und von dem anderen Ende den Sensor derart auf, dass sich die Lanzettenspitze und die Probeeintrittsöffnung während des Lanzettiervorgangs tangieren. Der Sensor wird in eine vorgesehene Vertiefung des Trägersubstrats verklebt, so dass die Sensoroberfläche und die Oberfläche des Trägersubstrats, zueinander eben sind. Darüber hinaus weist dieses Ende von oben nach unten ineinander übergehende und durchgehende Vertiefungen zur Aufnahme des kegelförmigen Dorns 1 auf. Die obere Vertiefung ist teilkreisförmig mit einem Durchmesser von 0,5 mm und einer Höhe von 0,3 mm und die untere Vertiefung ist kegelstumpfförmig mit einem Basisdurchmesser von 4,9 mm, einem Spitzendurchmesser von 0,5 mm und einer Höhe von 0,7 mm ausgebildet.

Das als Lanzette bezeichnete Spritzgussteil mit dem kegelförmigen Dorn wird an dem obenliegenden umlaufenden Rand der ausgesparten quadratischen Fläche auf der Unterseite des Trägersubstrats 12 formschlüssig verklebt.

Nach der Kontaktierung dieses Glucoseeinmalgebrauchssensors mit integrierter Lanzette mit einem potentiostatischen Messgerät, das für die geeignete Polarisationsspannung, Stromverstärkung, Messablaufsteuerung und Messwertanzeige sorgt, erfolgt die Probenahme und Ausmessung wie folgt: Der Nutzer legt den Finger, an dem die Probenahme erfolgen soll, fest in die obere Aussparung 13 des Trägersubstrates 12 und drückt mit dem Daumen der gleichen Hand von der Unterseite des Trägersubstrates 12 zentrisch so auf die Auswölbung der geprägten Kunststofffolie 10, dass diese sprunghaft nach oben ausweicht und damit auch die darüber angeordnete tiefste zentrische Auswölbung 3f nach oben bewegt. Diese vertikale Bewegung führt wiederum dazu, dass die im Querschnitt wellenkreisförmig ausgeformten Auswölbungen 3c,d der Fußfläche 3 nach oben sprunghaft und reversibel ausgeformt werden. Der darauf befindliche kegelförmige Dorn 1 bewegt sich sprunghaft nach oben und penetriert die Haut. Bei dieser vertikalen Bewegung stößt der kegelförmige Dorn mit dem Absatz 5 des zylinderförmigen Abschnitts an die Unterseite der Stirnfläche des Sensors 11 an, so dass gleichzeitig das Ende der Nut 8a auf der Oberfläche des kegelförmigen Dorns 1 die kapillare Probeeintrittsöffnung 11a des Sensors tangiert.

Bei entsprechender Eindringtiefe der Spitze 2 des kegelförmigen Dorns 1 in die Haut werden kapillarbluttransportierende Zellen verletzt. Das austretende Kapillarblut wird aufgrund der Kapillarkraftwirkung in der Nut 6 auf der Außenfläche des Dorns bis zum Ende der Nut 8a transportiert und sammelt sich dort an. Dadurch kann die Blutprobe von der kapillaren Probeeintrittsöffnung des Sensors übernommen und in die Messkammer des Sensors geleitet werden. Die zur Detektion erforderliche Probemenge beträgt zwischen 0,1 µl und 0,5 µl. Sobald die Messkammer gefüllt ist wird ein Triggerstrom erzeugt, der durch ein akustisches Signal dem Nutzer signalisiert, dass die Probenahme, die ca. 2 sec bis 5 sec dauert, beendet ist.

Wenn dann der Anwender Finger und Daumen von dem Glucoseeinmalgebrauchssensor mit integrierter Lanzette nimmt, gelangt die Auswölbung der geprägten Kunststofffolie 10 in ihre Ausgangsstellung.

Das Verhältnis der Auswölbungstiefe der nach oben und unten ausgeformten Auswölbungen 3c,d der Fußfläche 3 sowie die elastischen Materialeigenschaften der Auswölbungen sichern einerseits, dass bei Ausübung eines sprunghaften mechanischen Druckes die Wölbungen des Fußes 3 nach oben ausweichen und andererseits, dass bei Nachlassen des Drucks auf die Auswölbungen 3c,d diese in ihre ursprüngliche Lage zurückkehren.

Die Spitze des Dorns 2 wird dabei wieder innerhalb des Trägersubstrates versenkt.

### Beispiel 3

Erfindungsgemäßer Einmalgebrauchssensor mit integrierter Lanzette als Bestandteil einer Vorrichtung zum Nachweis von Glucose. Zur Erläuterung dienen Fig. 3 bis Fig. 5.

Der erfindungsgemäße Einmalgebrauchssensor mit integrierter Lanzette besteht im Einzelnen aus einer Lanzette, einem Sensor und einem Trägersubstrat, die zunächst separat voneinander hergestellt und im Anschluss formschlüssig integriert werden.

Die Lanzette des erfindungsgemäßen Einmalgebrauchssensors mit integrierter Lanzette wird wie in Beispiel 2 beschrieben aber unter Verzicht auf die plastische Kunststofffolie 10 hergestellt.

Der amperometrische Glucosesensor für den Einmalgebrauch wird wie in Beispiel 1 hergestellt, lediglich die Außenmaße sind auf 4 mm x 20 mm (BxL) geändert.
Zur formschlüssigen Integration von Lanzette und Sensor wird ein Trägersubstrat verwendet.

Das Trägersubstrat mit Außenmaßen von 4 mm x 25 mm x 2 mm (BxLxH) ist stufenartig ausgespart und nimmt von einem Ende die Lanzette und von dem anderen Ende den Sensor derart auf, dass sich die Lanzettenspitze und die Probeeintrittsöffnung während des Lanzettiervorgangs tangieren. Der Sensor 11 wird in eine vorgesehene Vertiefung des Trägersubstrats verklebt, so dass die Sensoroberfläche und die Oberfläche des Trägersubstrats 12, das die Lanzette von der Unterseite her aufnimmt, zueinander eben sind. Darüber hinaus weist dieses Ende von oben nach unten ineinander übergehende und durchgehende Vertiefungen zur Aufnahme des kegelförmigen Dorns 1 auf. Die obere Vertiefung 13 ist teilkreisförmig mit einem Durchmesser von 0,5 mm und einer Höhe von 0,3 mm und die untere Vertiefung 14 ist kegelstumpfförmig mit einem Basisdurchmesser von 4,9 mm, einem Spitzendurchmesser von 0,5 mm und einer Höhe von 0,7 mm ausgebildet.
Das als Lanzette bezeichnete Spritzgussteil mit dem kegelförmigen Dorn 1 wird an dem obenliegenden umlaufenden Rand der quadratisch ausgesparten Fläche auf der Unterseite des Trägersubstrats 12 formschlüssig verklebt.

Dieser erfindungsgemäße Einmalgebrauchsensor mit integrierter Lanzette zum Nachweis von Glucose ist Bestandteil einer Vorrichtung, in deren Gehäuse 15 gleichzeitig die Hard- und Software für die Signalverarbeitung und Messwertanzeige integriert ist.

In dieser Anwendungsform wird der Einmalgebrauchsensor mit integrierter Lanzette in einem Stapelmagazin 16 bevorratet. Ein Federmechanismus 17 im Gehäuse 15 sorgt dafür, dass die Sensoren im Stapelmagazin in Richtung einer Ausgangsführung federnd angedrückt werden. Ein unmittelbar neben dem Stapelmagazin angeordneter erster Aktor 18 schiebt über einen flachen Stößel auf Knopfdruck den unmittelbar am Ausgang des Stapelmagazins 16 befindlichen Sensor heraus in das Applikationsfenster 20, über das einerseits die obere teilkreisförmige Aussparung 13 des Trägersubstrates für den Nutzer von außen zugänglich wird und andererseits eine Kontaktierung des Sensors erfolgt und Messbereitschaft hergestellt wird. Wird nun der Finger, an dem die Probenahme erfolgen soll, in die obere Aussparung 13 des Trägersubstrates 12 gedrückt, wird von der Unterseite sprunghaft ein Stößel durch einen zweiten Aktor 19 auf die im Querschnitt kreiswellenförmigen Auswölbungen des Fußes 3c,d,f des kegelförmigen Dorns gedrückt, so dass die Auswölbungen des Fußes 3a in Richtung Spitze des Dorns ausweichen. Dadurch erfolgt eine sprunghafte vertikale Bewegung des kegelförmigen Dorns 1, wobei dieser mit dem Absatz 5 des zylinderförmigen Abschnitts an die Unterseite der Stirnfläche des Sensors stößt, so dass gleichzeitig das Ende der Nut 8a auf der Oberfläche des Dorns 1 bzw. die Austrittsöffnung des Kanals 8b die kapillare Eintrittsöffnung 11a des Sensors tangiert. Durch die vertikale Bewegung dringt die Spitze 2 des kegelförmigen Dorns in die Haut ein und verletzt bei entsprechender Eindringtiefe kapillarbluttransportierende Zellen. Die Nut 6 auf der Außenfläche des Dorns 1 transportiert aufgrund der Kapillarkraftwirkung interstitielle Flüssigkeit oder Kapillarblut bis zum Ende der Nut 8a und wird an dieser Stelle weiter über die kapillare Eintrittsöffnung des Sensors in die Messkammer des Sensors geleitet und dort detektiert. Die zur Detektion erforderliche Probemenge beträgt zwischen 0,1 µl und 0,5 µl. Nach der Probenahme, die über das Ansprechen des Sensors kontrolliert und akustisch bestätigt wird, zieht sich der Stößel des Aktors 19 zurück, so dass die gestreckten Auswölbungen 3c,d,f der Fußfläche 3 in ihre ursprüngliche Lage zurückkehren. Die Spitze des Dorns 2 wird dabei wieder innerhalb des Trägersubstrates 12 versenkt.

Der Anwender nimmt den Finger vom Einmalgebrauchssensor mit integrierter Lanzette zurück. Der Sensor wird durch eine weitere Stößelbewegung des ersten Aktors 18 ausgeworfen oder gelangt in ein Abfallsensormagazin 21 der Vorrichtung.

Es zeigen Fig. 1 bis Fig. 5:
- Fig. 1: Dreidimensional angeschnittene Darstellung eines Einmalgebrauchssensors mit integrierter Lanzette mit kegelförmigen Dorn 1, sprunghaft und reversibel verformbarer Fußfläche 3 mit nach unten eingebrachter wannenförmiger Vertiefung 3a, quaderförmiger Druckflächenauflage 3b, Sensor 11, Probeeintrittsöffnung 11a, Messkammer mit Drei-Elektrodenanordnung 11b, Trägersubstrat 12, Führungsschienen 12a,b, Querstrebe 12c, oberer teilkreisförmiger Aussparung 13, unterer kegelstumpfförmiger Aussparung 14 des Trägersubstrats 12

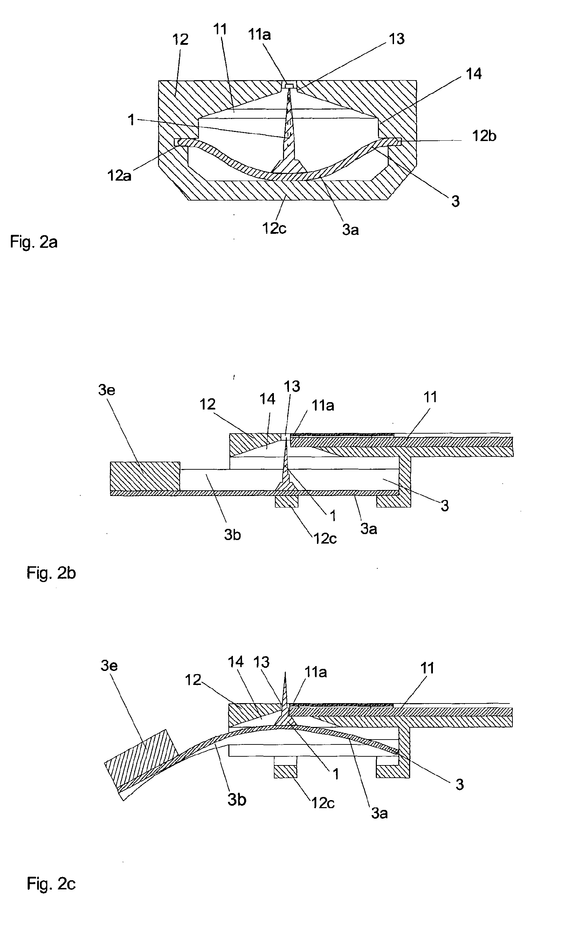

- Fig.2a: Schnittdarstellung eines Einmalgebrauchssensors mit integrierter Lanzette entlang der Querachse des kegelförmigen Dorns 1 im Ausgangszustand mit kegelförmigen Dorn 1, sprunghaft und reversibel verformbarer Fußfläche 3 mit nach unten eingebrachter wannenförmiger Vertiefung 3a, Sensor 11, Probeeintrittsöffnung 11a des Sensors 11, Trägersubstrat 12, Querstrebe 12c oberer teilkreisförmiger Aussparung 13, und unterer kegelstumpfförmiger Aussparung 14 des Trägersubstrats 12
- Fig.2b: Schnittdarstellung eines Einmalgebrauchssensors mit integrierter Lanzette entlang der Längsachse im Ausgangszustand mit kegelförmigem Dorn 1, sprunghaft und reversibel verformbarer Fußfläche 3 mit wannenförmiger runder Auswölbung 3a, freitragender Fußfläche 3b, quaderförmiger Druckauflagefläche 3e, Sensor 11, Probeeintrittsöffnung 11a des Sensors 11, Trägersubstrat 12, Querstrebe 12c, oberer teilkreisförmiger Aussparung 13 und unterer kegelstumpfförmiger Aussparung 14, des Trägersubstrats 12
- Fig. 2c: Schnittdarstellung eines Einmalgebrauchssensors mit integrierter Lanzette entlang der Längsachse im lanzettierenden Zustand mit kegelförmigen Dorn 1, sprunghaft und reversibel verformbarer Fußfläche 3 mit wannenförmiger runder Auswölbung 3a, freitragender Fußfläche 13b, quaderförmiger Druckauflagefläche 3e, Sensor 11, Probeeintrittsöffnung 11a des Sensors 11, Trägersubstrat 12, Querstrebe 12c, oberer teilkreisförmiger Aussparung 13, und unterer kegelstumpfförmiger Aussparung 14 des Trägersubstrats 12
- Fig. 3: Schnitt- und 3D-Darstellung des kegelförmigen Dorns entlang der längsseitigen Symmetrieachse des Einmalgebrauchssensors mit integrierter Lanzette mit Spitze 2, angeschnittener Fußfläche 3, zylinderförmigem Abschnitt 4, Absatz 5, Nut 6, Nutende, 8b und Kante 9
- Fig. 4: Schnittdarstellung der Lanzette entlang der längsseitigen Symmetrieachse des Einmalgebrauchssensors mit integrierter Lanzette im Ausgangszustand mit kegelförmigem Dorn 1, sprunghaft und reversibel verformbarer Fußfläche 3, im Querschnitt kreiswellenförmig nach oben und unten ausgeformten Auswölbungen 3c,d, tiefster Auswölbung 3f, reversibel und sprunghaft verformbarer Kunststofffolie 10, Sensor 11, Probeeintrittsöffnung 11a des Sensors, Trägersubstrat 12, oberer teilkreisförmiger Aussparung 13 und unterer kegelstumpfförmiger Aussparung 14 des Trägersubstrats 12

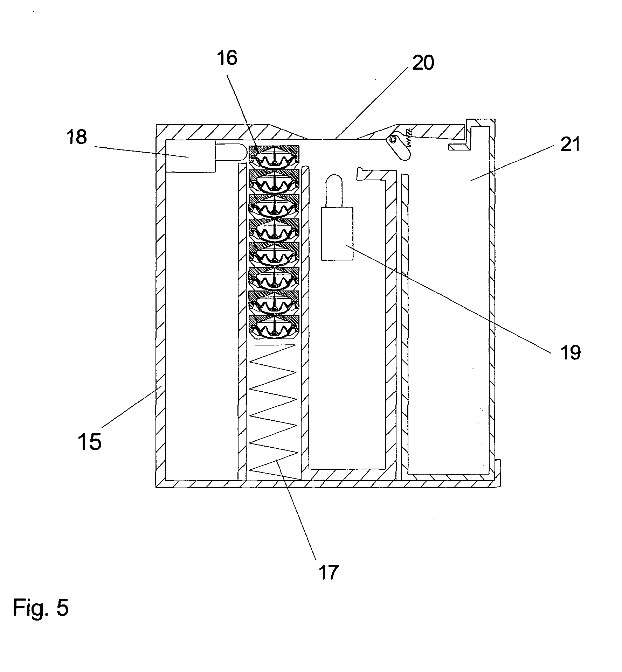

- Fig. 5: Prinzipdarstellung einer Vorrichtung mit Einmalgebrauchsensoren mit integrierter Lanzette mit Vorrichtungsgehäuse 15, Stapelmagazin 16, Federdruckvorrichtung 17, erstem Aktor 18, zweitem Aktor 19, Applikationsfenster 20 und Abfallmagazin 21

## Patentansprüche

1. Einmalgebrauchsmittel, umfassend einen Sensor und eine integrierte Lanzette für eine Lanzettierung von Haut zur Gewinnung einer Körperflüssigkeit, **dadurch gekennzeichnet, dass** die Lanzette einen kegelförmigen, formschlüssig angeordneten Dorn umfasst, der auf einer unter Druckbeaufschlagung sprunghaft und reversibel verformbaren Materialauswölbung positioniert ist und mindestens eine die Körperflüssigkeit aufnehmende, mit einer Reaktionsschicht des Sensors wirkverbindbare Nut und/oder einen Kanal aufweist.

2. Mittel nach Anspruch 1, **dadurch gekennzeichnet, dass** der Dorn symmetrisch oder zentrisch positioniert ist.

3. Mittel nach Anspruch 1 oder 2, **dadurch gekennzeichnet, dass** die Außenwandung des Dorns mindestens eine von der Spitze zum unteren Bereich sich in ihrer Tiefe vergrößernde Nut 6 und/oder im Inneren von der Spitze bis in den unteren Bereich führenden Kanal 7 aufweist, der im unteren Bereich an einer Stelle der Außenwandung austritt, wobei das Ende der Nut 8a und/oder die Austrittsöffnung 8b des Kanals 7 mit der kapillaren Eintrittsöffnung 11a des Sensors über eine formschlüssige Verbindung wirkverbindbar ist.

4. Mittel nach einem der vorhergehenden Ansprüche, **dadurch gekennzeichnet dass** die Lanzette aus einem kegelförmigen Dorn 1 und einer Fußfläche 3 mit einer oder mehreren unter mechanischem Druck sprunghaft und reversibel verformbaren Materialauswölbungen 3a, 3c, d, f, besteht und auf der der kegelförmige Dorn 1 formschlüssig symmetrisch oder zentrisch positioniert ist, dessen Außenwandung von der Spitze 2 bis in den unteren Bereich eine oder mehrere sich in ihrer Tiefe vergrößernde Nuten 6 oder im Inneren von der Spitze bis in den unteren Bereich einen inneren Kanal 7 aufweist, der im unteren Bereich an einer Stelle der Außenwandung austritt und bei dem das Ende der Nut 8a oder die Austrittsöffnung 8b des inneren Kanals 7 mit der kapillaren Eintrittsöffnung 11a des Einmalgebrauchssensors 11 über eine formschlüssige Verbindung zu einem Trägersubstart 12 während des Lanzettiervorgangs durch eine sprunghafte, reversible Verformung der Materialauswölbungen 3a, 3c, d 3f der Fußfläche 3, die durch mechanischen Druck ausgelöst wird, in Kontakt gebracht werden.

5. Mittel nach einem der vorhergehenden Ansprüche, **dadurch gekennzeichnet, dass** die Nut oder Nuten 6 auf der Außenfläche des kegelförmigen Dorns 1 zwischen 20 µm und 180 µm tief sind und vorzugsweise einen teilkreisförmigen Querschnitt aufweisen.

6. Mittel nach einem der vorhergehenden Ansprüche, **dadurch gekennzeichnet, dass** die Fußfläche 3 aus einer rechteckigen dünnen und plastischen Folie mit einer Breite zwischen 4 mm und 10 mm, einer Länge zwischen 10 mm bis 50 mm und mit einer Dicke zwischen 0,1 mm und 0,5 mm besteht, die entlang der symmetrischen Längsachse eine runde wannenförmige nach unten weisende Auswölbung 3a mit einer Tiefe zwischen 1 mm und 2 mm und einer Breite zwischen 3 mm und 7 mm und im ersten Drittel der Vertiefung zentrisch und nach oben weisend einen kegelförmigen Dorn 1 aufweist.

7. Mittel nach einem der vorhergehenden Ansprüche, **dadurch gekennzeichnet, dass** die Fußfläche 3 an seinen Rändern im ersten Drittel links und rechts bis zur virtuellen Querachse des kegelförmigen Dorns 1 oben und unten in Führungsschienen 12a, b des Trägersubstrats 12 fixiert wird, durch eine zusätzliche Querstrebe 12c des Trägersubstrats 12, die entlang der Querachse des kegelförmigen Dorns 1 verläuft, an der tiefsten Stelle der runden Auswölbung tangiert wird und ihre freitragende Restfläche 3b über das Ende des Trägersubstrates 12 hinaus ragt.

8. Mittel nach einem der vorhergehenden Ansprüche, **dadurch gekennzeichnet, dass** die Fußfläche 3 mit einer quadratischen Grundfläche zwischen 4 mm und 10 mm eine oder mehrere sprunghaft und reversibel verformbare im Querschnitt kreiswellenförmig geschlossene oder segmentartig ausgeformte Auswölbungen (3c, d, f) nach oben und unten aufweist und mit einer plastisch verformbaren Kunststofffolie 10, die entgegen der Kegelrichtung ausgewölbt ist, mit dem ebenen, rechteckigen Rand der Fußfläche 3 formschlüssig verbunden ist.

9. Mittel nach einem der vorhergehenden Ansprüche, **dadurch gekennzeichnet, dass** der kegelförmige massive Dorn 1 zwischen 1 mm und 3 mm hoch ist, eine Spitze 2 und eine sprunghaft und reversibel verformbare Fußfläche 3 aufweist.

10. Mittel nach einem der vorhergehenden Ansprüche, **dadurch gekennzeichnet, dass** der kegelförmige massive Dorn 1 im unteren Drittel einen zylinderförmigen Abschnitt 4 mit einem Durchmesser zwischen 0,2 mm und 1,0 mm und einer Höhe zwischen 0,2 mm und 0,5 mm aufweist, der nach einem Absatz 5 bis zur Fußfläche 3 kegelförmig ausläuft.

11. Mittel nach einem der vorhergehenden Ansprüche, **dadurch gekennzeichnet, dass** die in der Außenwandung des kegelförmigen massiven Dorns 1 von der Spitze bis zum Beginn des kegelförmigen Abschnitts 4 verlaufende Nut oder die verlaufenden Nuten 6 vorzugsweise einen teilkreisförmigen Querschnitt mit einem Durchmesser zwischen 5 µm und 180 µm aufweisen und sich von der Spitze zum unteren Bereich des Dorns in ihrer Tiefe und Breite oder im Durchmesser ihres teilkreisförmigen Querschnitts vergrößern.

12. Mittel nach einem der vorhergehenden Ansprüche, **dadurch gekennzeichnet, dass** der kegelförmige massive Dorn 1 einen inneren Kanal 7 mit einem Durchmesser von 5 bis 180 µm von der Spitze bis zu einer seitlichen Austrittsöffnung 8b über dem kegelförmigen Abschnitt 4 aufweist.

13. Mittel nach einem der vorhergehenden Ansprüche, **dadurch gekennzeichnet, dass** die Positionierung und formschlüssige Aufnahme von Lanzette und Sensor mittels eines in Längsrichtung stufenförmig ausgesparten Trägersubstrats 12 erfolgt.

14. Mittel nach einem der vorhergehenden Ansprüche, **dadurch gekennzeichnet, dass** der kegelförmige Dorn 1 und der Sensor 11 räumlich derart zueinander angeordnet werden, dass bei der sprunghaften reversiblen Verformung der Materialauswölbung 3a, c, d, f der Fußfläche 3 die vertikale Bewegung des kegelförmigen Dorns 1 aufgrund des Absatzes 5 unterhalb der Fläche an der Stirnseite des Sensors 11 begrenzt wird und auf diese Weise das Ende einer der Nuten 8a bzw. die Austrittsöffnung 8b des Kanals 7 reproduzierbar auf die gleiche Höhe mit der kapillaren Eintrittsöffnung 11a des Sensors 11 (Probeeintrittsöffnung) gebracht werden und sich tangieren.

15. Mittel nach einem der vorhergehenden Ansprüche, **dadurch gekennzeichnet, dass** die Probeeintrittsöffnung 11a des Sensors 11 einen Docht zur erleichterten Probeübernahme enthält.

16. Mittel nach einem der vorhergehenden Ansprüche, **dadurch gekennzeichnet, dass** der Einmalgebrauchssensor mit integrierter Lanzette, der als Fußfläche 3 eine oder mehrere reversibel verformbare im Querschnitt kreiswellenförmig geschlossene oder segmentartig ausgeformte Auswölbungen 3c, d, f nach oben und unten aufweist, in magazinierter Form 16 Bestandteil einer Vorrichtung ist, in deren Gehäuse 15 gleichzeitig die Hard- und Software für die Signalverarbeitung und Messwertanzeige integriert ist und deren Aktorik 18, 19 die Bereitstellung des Sensors, die sprunghafte Auslösung der Fußflächenverformung und die Sensorverwerfung übernimmt.

17. Mittel nach einem der vorhergehenden Ansprüche, **dadurch gekennzeichnet, dass** alle beschriebenen Teile aus Kunststoffen mittels Mikrospritzguss, Heißprägetechnik und in Kombination mit Laserablation gefertigt werden.

18. Mittel nach einem der vorhergehenden Ansprüche, **dadurch gekennzeichnet, dass** als Materialien für die beschriebenen Teile Folien oder Platten aus Polycarbonat, Polymethylmethacrylat, Polystyrol, Polyether, Polyethylenterephterphtalat, Acrylnitril-Butadien-Styrol-Copolymer, Polyvinylchlorid, ein thermoplastisches Elastomer oder Kombinationen davon mit Dicken zwischen 0,1 mm und 4,0 mm verwendet werden.

19. Verfahren zur Herstellung eines analytischen Einmalgebrauchsmittels nach einem der Ansprüche 1 bis 18, **dadurch gekennzeichnet, dass** Sensor und Lanzette separat gefertigt werden und folgend in planarer Form kraft- und formschlüssig durch ein Trägersubstrat miteinander verbunden werden.

20. Verfahren nach dem vorhergehenden Anspruch, **dadurch gekennzeichnet, dass** die Einzelbestandteile des Sensors und der Lanzette aus Kunststoffen mittels Mikrospritzguss, Heißprägetechnik und/oder Tiefziehverfahren hergestellt werden.

21. Verfahren nach einem der Ansprüche 19 oder 20, **dadurch gekennzeichnet, dass** der Kunststoff Polycarbonat, Polymethylacrylat, Polysterol, Polyether, Polyethylenterephterphtalat, Acrylnitril-Butadien-Styrol-Copolymer, Polyvinylchlorid, thermoplastische Elastomere und/oder Kombinationen aus diesen umfasst.

22. Verwendung des analytischen Einmalgebrauchsmittels für die Lanzettierung der bzw. durch die Haut.

23. Verwendung nach dem vorhergehenden Anspruch, **dadurch gekennzeichnet, dass** eine Körperflüssigkeit entnommen wird.

24. Verwendung nach Anspruch 22 oder 23, **dadurch gekennzeichnet, dass** in der entnommenen Körperflüssigkeit die Glucose- und/oder Laktatwerte bestimmt werden.
